# EUROPEAN PATENT SPECIFICATION

(11) **EP 0 642 357 B1**
(45) Date of publication and mention of the grant of the patent: **25.07.2001**
(21) Application number: 93904844.3
(22) Date of filing: 04.02.1993
(51) Int. Cl.: A61K 51/00, C07K 7/00, C07K 7/06, C07D 213/40

(54) **RADIOLABELLED SOMATOSTATIN**
RADIOAKTIV MARKIERTE SOMATOSTATIN
COMPOSES DE SOMATOSTATINE RADIOMARQUES

(30) Priority: 05.02.1992 US 831724; 05.02.1992 US 831780; 04.02.1993 US 13527
(43) Date of publication of application: 15.03.1995
(60) Divisional application: 00204594.6
(73) Proprietor: MALLINCKRODT INC., St. Louis, MO 63134 (US)
(72) Inventor: LYLE, Leon, Webster Groves, MO 63119 (US); RAJAGOPALAN, Raghavan, Maryland Heights, MO 63043 (US); DEUTSCH, Karen, Maryland Heights, MO 63043 (US); DUNN, Thomas Jeffrey, Cedar Hill, MO 63016 (US); SRINIVASAN, Ananthachari, St. Charles, MO 63304 (US); VANDERHEYDEN, J.L., St. Louis, MO 63131 (US)
(74) Representative: Jones, Alan John
(86) International application number: US9300939
(87) International publication number: WO9315770

(56) References cited:
- EP-A- 0 333 356
- EP-A- 0 421 366
- WO-A-90/15818
- WO-A-92/05154
- WO-A-92/21383
- WO-A-93/15771
- GB-A- 2 225 579
- US-A- 4 746 505
- US-A- 4 746 507
- US-A- 4 837 003
- US-A- 4 965 392
- US-A- 5 037 630
- US-A- 5 187 264
- Inorganic Chemistry, Volume 29, No. 16, August 1990 (ACS, Easton, PA USA), BRYSON et al., "Protecting Groups in the Preparation of Thiolate Complexes of Technetium", pp. 2948-2951, see especially page 2950.
- Journal of Nuclear Medicine, Volume 32, Number 10, October 1991, KWEKKEBOOM et al., "Radioiodinated Somatostatin Analog Scintigraphy in Small-Cell Lung Cancer", pp. 1845-1848.
- Journal of Nuclear Medicine, Volume 32, No. 6, June 1991, LARSON, "Receptors on Tumors Studied with Radionuclide Scintigraphy", pages 1189-1191.
- Seventh International Symposium on Radiopharmacology, (1991) COX et al., "Technetium Labelled Somatostatin: A Potential Agent for In Vivo Tumor Localisation", page 16.
- Journal of Nuclear Medicine, Volume 32, No. 5, May 1991, KWEKKEBOOM et al., "(ln-111-DTPA-D-Phe)- Octreotide Suntigraphy in Neuro- Endocrine Tumors", Abstract No. 305, page 981.
- Tetrahedron Letters, Volume 30, No. 15 (1989), MISRA et al., "Synthesis of a Novel Diaminodithiol Ligand for Labeling Proteins and Small Molecules with Technetium-99M", pages 1885-1888.

## Description

### FIELD OF THE INVENTION

This invention relates generally to novel ligands and compounds for use in diagnostic tissue imaging and more particularly, to site specific radiolabelled peptides, to novel ligands for preparing radiolabelled compositions, to methods of preparing such site specific radiolabelled peptides, and to pharmaceutical compositions comprising these site specific radiolabelled peptides for diagnostic imaging or therapeutic use.

### BACKGROUND OF THE INVENTION

Scintigraphic imaging and similar radiographic techniques for visualizing tissues in vivo are finding ever-increasing application in biological and medical research and in diagnostic and therapeutic procedures. Generally, scintigraphic procedures involve the preparation of radioactive agents which, upon introduction to a biological subject, become localized in the specific organ, tissue or skeletal structure of choice. When so localized, traces, plots or scintiphotos depicting the in vivo distribution of radiographic material can be made by various radiation detectors, e.g., traversing scanners and scintillation cameras. The distribution and corresponding relative intensity of the detected radioactive material not only indicates the space occupied by the targeted tissue, but also indicates a presence of receptors, antigens, aberrations, pathological conditions, and the like.

In general, depending on the type of radionuclide and the target organ or tissue of interest, the compositions comprise a radionuclide, a carrier agent designed to target the specific organ or tissue site, various auxiliary agents which affix the radionuclide to the carrier, water or other delivery vehicles suitable for injection into, or aspiration by, the patient, such as physiological buffers, salts, and the like. The carrier agent, i.e. ligand, attaches or complexes the radionuclide to the peptide carrier agent, which results in localizing the radionuclide being deposited in the location where the carrier agent concentrates in the biological subject.

Technetium-99m(^{99m}Tc) is a radionuclide which is widely known for its uses in tissue imaging agents. Due to its safety and ideal imaging properties, this radionuclide is conveniently available commercially in the oxidized pertechnetate form (^{99m}TcO₄⁻) hereinafter "pertechnetate-Tc99m". However, pertechnetate will not complex with the most commonly used biological carriers for radionuclide tissue imaging. Thus, technetium-labelled imaging agents are generally prepared by admixing a pertechnetate-Tc99m isotonic saline solution, a technetium reductant (reducing agent) such as stannous chloride or sodium dithionite, and a chelate conjugated to the desired peptide carrier agent for targeting the organ of interest. Alternatively, a transfer ligand may be added to the reduced pertechnetate prior to addition to the chelate-biological molecule to maintain the oxidation state within a desired level. Examples of such include 99m Tc-tartrate or 99m Tc-gluconate.

Another problem is that technetium-containing scintigraphic imaging agents are known to be unstable in the presence of oxygen, primarily since oxidation of the reductant and/or the technetium -99m destroys the reduced technetium -99m/targeting carrier complex. Accordingly, such imaging agents are generally made oxygen-free by saturating the compositions with oxygen-free nitrogen gas or by preparing the agents in an oxygen-free atmosphere. Stabilization of imaging agents can also be achieved through chemical means. U.S. Patent Number 4,232,000, Fawzi, issued November 4, 1980, discloses the use of gentisyl alcohol as a stabilizer for technetium imaging agents. Similarly, U.S. Patent Number 4,233,284, Fawzi, issued November 11, 1980 discloses the use of gentisic acid as a stabilizer.

### SUMMARY OF THE INVENTION

The present invention discloses novel ligands which are useful in preparing radiolabelled compositions. The ligands have been found to be particularly useful in labelling under a wide variety of acidic to basic labelling conditions.

The present invention further discloses novel radiolabelled peptide compounds, methods of preparing these compounds, pharmaceutical compositions comprising these compounds and the use of these compounds in kits for the therapeutic treatment of tumors and for diagnostic imaging of tumors. Certain tumors of endocrine-origin contain large numbers of receptors having a high affinity for somatostatin. Krenning, et al. (Lancet 8632, 242-244 (1989)). Examples of such tumors, having large numbers of high-affinity somatostatin receptors, are pituitary tumors, central nervous system tumors, breast tumors, gastro-entero-pancreatic tumors, small cell carcinoma of the lung, lymphomas, as well as their metastases.

In diagnostic tumor localization, a radiolabelled compound must be easily detectable and highly selective. High selectivity, which is essential in these compounds means that the diagnostic compound, after having been introduced into the body, accumulates to a greater degree in the target tissue or tissues, i.e. a malignant tumor, than in surrounding tissues. In using somatostatin or other such peptides as carrier agents in radiolabelled compounds, the specific high selectivity of the particular peptide used provides for the strong accumulation of the diagnostic/therapeutic compound in the target tissue or tissues, such as in tumors in the case of somatostatin, compared with the concentration thereof in non-target tissues. Additionally, therapeutic treatment of malignant tumors is achieved when radiolabelled peptide compounds are constructed using high energy Beta or Alpha emitting isotopes rather than the pure gamma emitters customarily used for diagnostic purposes.

The radiolabelled peptide compounds of the present invention employ the somatostatin peptide: 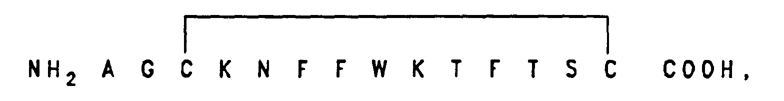 wherein A represents Alanine, G represents Glycine, C represents Cysteine, K represents Lysine, N represents asparagine, F represents phenylalanine, W represents tryptophan, T represents threonine, and S represents serine, or a suitable derivative thereof.

In targeting particular receptors with radiolabelled somatostatin, it is not necessary that the complete fourteen (14) residue sequence of somatostatin be present. Binding is thought to reside primarily in the central core portion of the molecule, primarily, the phenylalanine-tryptophan-lysine-threonine or F W K T sequence. Through substitution in the somatostatin sequence, including some limited substitutions in the central core portion and perhaps incorporating (d) amino acid enantiomorphs, additional useful peptides are developed without affecting the binding specificity and affinity desired. Likewise peptidomimetic molecules may be prepared to duplicate this specific binding function. An example of such a useful peptide is SANDOSTATIN™ manufactured by SANDOZ Pharmaceuticals, Ltd., Basel Switzerland. The sequence of the Sandostatin™ peptide is: 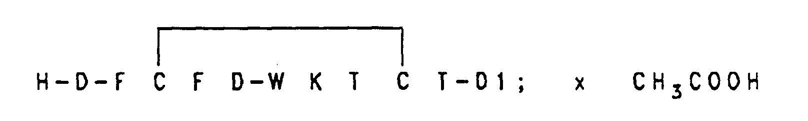 wherein x equals 1.4 to 2.5

In the present invention, the somatostatin peptide itself, or a molecule having somatostatin receptor specificity, may be radiolabelled using more than one method. The reaction generally takes place between the amino groups in the peptide and the carbonyl group in the active ester to form an amide bond. In particular, the peptides can be radiolabelled using either a conventional method referred to as "post-formed chelate approach" or by a recent method referred to as "pre-formed chelate approach" developed by Fritzberg et al., U.S. Patents Numbers 4,965,392 and 5,037,630 incorporated herein by reference. In the "pre-formed approach," the desired ligand is complexed with the radionuclide and then conjugated to somatostatin or a molecule having somatostatin receptor specificity. In the "post-formed approach," the desired ligand is first conjugated to the peptide and the resulting conjugate is incubated with 99mTc sodium pertechnetate solution obtained from ⁹⁹Mo/^{99m}Tc generator along with a reducing agent. In the present invention, the latter approach has the additional advantage of allowing preparation of the complex in kit form. Users merely add Na^{99m}TcO₄ to the ligand-somatostatin conjugate or a derivative thereof for labelling to occur.

It is important to note a unique aspect of the present invention whereby the desired conjugation reaction should be directed to occur only at the alpha amino group of the somatostatin. The critical binding region for the peptide includes the amino group at the lysine position, or K. Conjugation at the alpha amino group is important so as to avoid interference with the binding specificity of the somatostatin peptide. It is therfore necessary to take steps to assure that the conjugation reaction takes place at the alpha amino group. The desired conjugation reaction will take place most readily when the alpha amino group is in the "free base" form, i.e., deprotonated to the NH₂ form, while the epsilon amino group is protonated, i.e., in the NH₃⁺ form. Therefore, according to the present invention, it is important to perform the conjugation reaction at neutral pH or within the range of pH 7.0 to pH 9.5. Performing the conjugation reaction at such a pH deactives the lysine position relative to the alpha amino group, and protonates the more basic epsilon amino group, thus making the epsilon amino group less reactive. In other words, by carrying out the conjugation reaction at the pH noted above, deprotonation of the epsilon amino group is prevented and conjugation occurs at the desired site of the alpha amino group.

Using either method of labelling the somatostatin peptide, the ligand can be used to incorporate the preferred radionuclide metal ion such as technetium, rhenium, indium, gallium, samarium, holmium, yttrium, copper, or cobalt.

The above-described unique characteristics of the present invention make radiolabelled somatostatin and its derivatives very attractive for diagnostic purposes as well as for radiotherapy. The compounds of the present invention may be labelled with any radionuclide favorable for these purposes. Such suitable radionuclides for radiotherapy include but are not limited to Re-186, copper-67, Re-188 and cobalt-60. For diagnostic purposes the most suitable radionuclides include but are not limited to the transition metals as exemplified by technetium-99m and copper-62.

Due to the unique mechanism employed in the present invention to label the alpha amino group of somatostatin and avoid the epsilon amino group(s) (which would inhibit the ability of somatostatin peptides to bind to its receptor) a significantly advantageous radiolabelled peptide compound for radiotherapy and diagnostic imaging of tumors is achieved.

It is therefore an object of the present invention to provide a selective agent, both for the diagnostic imaging and for the therapeutic treatment of tumors containing high-affinity somatostatin receptors having a significantly high target to background ratio.

### DETAILED DESCRIPTION OF THE INVENTION

The novel ligands according to the invention are defined according to the general formula given in claim 1.

The novel ligands described above, may be incorporated into radionuclide complexes used as radiographic imaging agents. Further, these ligands or complexes can be covalently or non-covalently attached to biologically active carrier molecules, such as, antibodies, enzymes, peptides, peptidomimetics, hormones, and the like. The complexes of the present invention are prepared by reacting one of the aforementioned ligands with a radionuclide containing solution under radionuclide complex forming reaction conditions. In particular, if a technetium agent is desired, the reaction is carried out with a pertechnetate solution under technetium 99m complex forming reaction conditions. The solvent, if other than water or saline, may then be removed by any appropriate means, such as evaporation. The complexes are then prepared for administration to the patient by dissolution or suspension in a pharmaceutically acceptable vehicle.

One peptide employed in the present invention is a somatostatin peptide or derivatives thereof as described in U.S. Patent Number 4,395,403.

The somatostatin peptide may be radiolabelled using a pre-formed or post-formed methodology. In a preferred embodiment according to the present invention, the somatostatin or a molecule having somatostatin receptor specificity, is conjugated with the ligand illustrated in Figure 1. 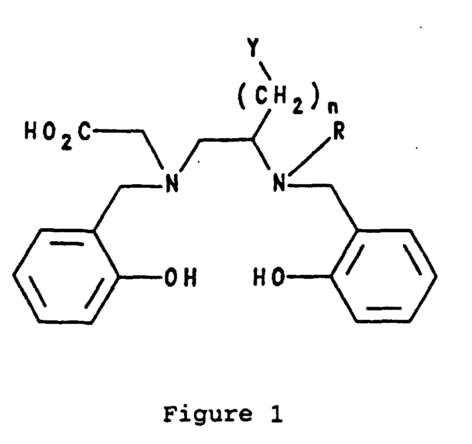 wherein n varies from 1 to 10, and Y is a coupling moiety selected from the group consisting of carboxyl, amino, isocyanate, isothiocyanate, imidate, maleimide, chlorocarbonyl, chlorosulfonyl, succinimidoloxycarbonyl, haloacetyl, and C₁₋₁₀ N-alkoxycarbamoyl such as N-methoxycarbamoyl and t-butoxycarbamoyl -whereby N-methoxycarbamoyl⁻ is preferable; and R is selected from the group consisting of hydrogen and C₁₋₁₀ alkyl such as methyl and t-butyl -whereby methyl is preferable.

In another preferred embodiment, the somatostatin or a molecule having somatostatin receptor specificity, can be conjugated with a metal complex as shown in Figure 2. 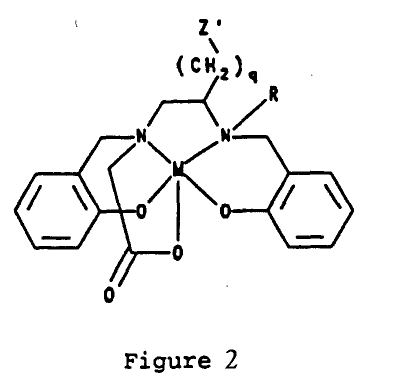 wherein Z', q and R are defined the same respectively as Y, n and R of Figure 1 and M is a radionuclide suitable for diagnostic imaging or therapeutic use such as technetium, rhenium, copper, cobalt, indium, gallium, samarium, yttrium and holmium.

Common esters which have been found useful in this labelling technique are o- and p- nitrophenyl, 2- chloro-4-nitrophenyl, cyanomethyl, 2-mercaptopyridyl, hydroxybenztriazole, N-hydroxysuccinimide, trichlorophenyl, tetrafluorophenyl, thiophenyl, tetrafluorothiophenyl, o-nitro-p-sulfophenyl, N-hydroxyphthalimide and the like. For the most part, the esters will be formed from the reaction of the carboxylate with an activated phenol, particularly, nitro-activated phenols, or a cyclic compound based on hydroxylamine.

The choice or an appropriate sulfur protecting group is essential to achieving the maximum utility form the invention. The protecting groups are displaced from the compound during the labelling in what is believed to be a metal-assisted cleavage: i.e., the protective groups are displaced in the presence of a radionuclide during the labelling process. The radiolabelling procedure thus is simplified, which is a significant advantage when the chelating compounds are to be radiolabelled in a hospital laboratory shortly before use. Additionally, another advantage of the present invention is that extreme basic pH conditions and harsh conditions associated with certain known radiolabeling procedures or procedures for removal of other sulfur protected groups are avoided. Thus, both base-sensitive and acid-sensitive groups on the chelating compounds survive the radio-labelling step intact. Suitable sulfur-protecting groups, when taken together with the sulfur atom to be protected, include hemithioacetal groups such as ethoxyethyl, tetrahydrofuranyl, methoxymethyl, and tetrahydropyranyl. Other suitable sulfur protecting groups are C₁₋₂₀ acyl groups, preferably alkanoyl, benzoyl, and C₁₋₂₀ alkoxy-carbonyl groups, preferably N-methoxycarbonyl and t-butoxycarbonyl. Other possible formulas for the chelating compounds are described in the European Patent Application assigned publication number 0 284 071.

Synthesis of the Tc-99m bifunctional chelate and subsequent conjugation to a somatostatin peptide, or a derivative thereof, can be performed as described in the European Patent Application assigned publication number 0 284 071 and U.S. Patent Number 4,965,392 and related technologies as covered by U.S. patent numbers 4,837,003, 4,732,974 and 4,659,839.

After purification, technetium-99m labelled somatostatin peptide, or derivatives thereof, may be injected into a patient for diagnostic imaging or therapeutic use. The technetium-99m somatostatin compound is capable of reliably visualizing tumors within minutes of post-injection. The somatostatin peptide when radiolabelled with the technetium-99m triamide thiolate bifunctional chelate is efficacious as an in vivo diagnostic agent for the imaging of tumors of the type described above.

The ligands of the present invention may be prepared from commercially available starting materials such as 2-(2-aminoethyl)pyridine, 2-aminomethyl pyridine, lysine, glutamic acid, aminoadipic acid, mercaptoacetic acid, etc. by standard synthetic methods as described in the following Examples.

### Example 1

### Preparation of 2-aza-4-[N-(S-benzoyl)mercaptoacetyl-8-[N-(t-butoxy)carbonyl]amino-3-oxo-1-(2-pyridyl)octane.

A mixture of 4-amino-2-aza-8-[N-(t-butoxy)carbonyl]-amino-3-oxo-1-(2-pyridyl)octane (1.70 g, 5 mmol) and N-[(S-benzoyl)mercapto]acetoxy-succinimide (1.53 g, 5.5 mmol) in acetonitrile (15 mL) was stirred at ambient temperature for 4 hours. The reaction mixture was poured onto water (100 mL) and kept at 4 to 8 °C (refrigerator) for about 16 hours. The precipitate was collected by filtration, washed well with water, dried, and recrystallized from acetonitrile to give 1.2 g of colorless solid, mp 133-135 °C. Anal. Calcd. for C₂₅H₃₄N₄0₅S: C, 60.70; H, 6.61; N, 10.89; S, 6.26. Found: C, 60.79; H, 6.65; N, 10.91; S, 6.30.

### Example 2

### Preparation of 2-aza-4-[N-(S-tetrahydropyranyl)-mercapto] acetyl-8-N-(t-butoxy)carbonyl] amino-3-oxo-1-(2-pyridyl)-octane.

A mixture of 4-amino-2-aza-8-[N-(t-butoxy)carbonyl)]-amino-3-oxo-1-(2-pyridyl)octane (3.36 g, 10 mmol) and N-[(S-tetrahydropyranyl)mercapto-acetoxy]-succinimide (2.40 g, 10 mmol) in acetonitrile (25 mL) was stirred at ambient temperature for 4 hours. The reaction mixture was poured onto water (100 mL) and extracted with methylene chloride (3 x 25 mL). The combined organic extracts were washed with water, dried (MgSO₄), filtered, and the filtrate taken to dryness under reduced pressure. The gummy residue was chromatographed over silica gel (200 g) using chloroform/methanol (95:5) as eluent to give 3.2 g of off-white solid, mp 87-90 °C. 13C-NMR (CDCl₃) δ 171.8, 171.7, 170.0, 156.9, 156.7, 156.3, 149.3, 137.0, 122.5, 121.9, 84.0, 83.6, 79.0, 66.2, 65.7, 53.2, 44.4, 44.3, 40.0, 35.0, 34.6, 31.7, 31.0, 29.4, 28.2, 25.0, 24.9, 22.4, 21.9, 21.6.

### Example 3

### Preparation of 6-aza-4-[N-(S-benzoyl)mercapto]acetyl-5-oxo-7-(2-pyridyl)-heptanoic acid.

A mixture of t-butyl 6-aza-4-[N-(S-benzoyl)-mercapto]-acetyl-5-oxo-7-(2-pyridyl)heptanoate (2.35 g, 5 mmol) and trifluoroacetic acid (5 mL) was kept at ambient temperature for 1 hour. The solution was then poured onto ether (100 mL). The precipitate was then collected by filtration, washed well with ether, and dried to yield 1.5 g of off white solid. ¹H-NMR (DMSO-d₆) δ 8.49-8.71 (m, 3H), 7.85-8.00 (m, 3H), 7.60-7.70 (m, 1H), 7.40-7.60 (m, 4H), 4.45 (d, 2H), 4.31 (m, 1H), 3.87 (dd, 2H), 2.27 (m, 2H), 1.95 (m, 1H), 1.80 (m, 1H). ¹³C-NMR (DMSO-d₆) δ 191.1, 174.4, 172.0, 167.7, 157.5, 146.7, 140.3, 136.3, 134.5, 129.5, 127.2, 123.5, 122.5, 52.7, 42.9, 32.6, 30.1, 27.0. FAB mass spectrum, m/Z 416 (M + 1).

### Example 4

### Preparation of 7-aza-5-N-[(5-benzoyl)mercapto]acetyl-1-N-(t-butoxy-carbonyl)amino-6-oxo-9-(2-pyridyl)nonane.

A mixture of N-t-BOC-lysine-2-(2-pyridyl)ethylamide (1.75 g, 5 mmol) and N-[(5-benzoyl)mercapto]acetoxysuccinimide (1.53 g, 5.5 mmol) in acetonitrile (15 mL) was stired at ambient temperature for four hours. The reaction mixture was poured onto water (100 ml) and cooled in ice-salt bath for two hours. The precipitate was collected by filtration, washed with water, dried, and recrystalized from acetronitrile to give 2.3 g (88 %) of colorless solid. m.p. 138-140 C. Anal. Calcd. for C₂₆H₃₆N₄O₅S: C, 61.36; H, 7.27; N, 10.67; S, 6.10. Found: C, 61.39; H, 7.18; N, 10.62; S, 6.01.

### Example 5

### Preparation of 10-[(S-tetrahydropyranyl)mercapto]acetamido-5,12-diaza-4,11-dioxo-13(2-pyridyl)tridecanoic acid.

A mixture of 4-(4-amino)butyl-3,6-diaza-2,5-dioxo-1-(S-tetrahydropyranyl)mercapto-7-(2-pyridyl)heptane (790 mg, 2.0 mmol) and S-tetrahydropyranylmercaptoacetic acid (220 mg, 2.2 mmol) in acetonitrile (10 mL) was heated under reflux for four hours and stired at ambient temperature for sixteen hours. The solvent was removed under reduced pressure and the residue was purified by flash chromatography over reverse phase (25 g) eluted with water followed by methanol/water (1:1). Evaporation of the solvent afforded the desired ligand (510 mg) as colorless, amorphous solid. Anal. Calcd. for C₂₃H₃₄N₄O₆S x 0.33 H₂O: C, 55.20; H, 6.93; N, 11.20; S, 6.40: H₂O, 1.20. Found: C, 54.81; H, 6.99; N, 11.18; S, 6.39: H₂O, 1.19. Mass spectrum (thermospray) M/Z 495 (M + 1).

As noted above, the choice of protecting groups for the ligands according to the present invention has been found to be important. In particular, finding the proper protecting group for protection of the sulfur moiety has created difficulty in past ligand technology. It has been discovered that the use of hemithioacetal protecting groups such as tetrahydropyrannyl (THP) are especially useful during the labelling procedures.

Labelling of pyridine ligands as described above having a hemithioacetal protecting group has been carried out as shown in the following examples.

### Example 6

Preparations were made as follows:

To 0.1 mL stannous gluconate (from a lyophilized kit containing 50 mg sodium gluconate and 1.2 mg stannous chloride, and reconstituted with 1.0 mL of degassed water) was added 1.0 mL pertechnetate, Tc-99m (about 3 mCi). The above is allowed to stand for 5 min at room temperature, before it is adjusted for pH with either HCl or NaOH (target Ph were 5, 6, 7 and 8). 0.12 mL of a pyridine ligand (SN₂Py) (0.88 mg/mL, 33% IPA/water) was then added. The preparations were incubated in a boiling water bath for 5 minutes.

An aliquot of the preparation was injected on an HPLC (C18 reverse phase), and the results of the radioactive profiles were integrated. Radiolabelling yields (RCY) are expressed as a percent of the peak of interest (Tc-99m SN₂Py). Recovery studies were performed by measuring the amount of activity injected on the system vs recovered. The pH of the preparations were also measured with a pH electrode.

### Example 6: Results

| Target pH | RCY | Recovery (%) | Measured pH |
|---|---|---|---|
| 5 | 43.1 | 90 | 5.1 |
| 6 | 53.6 | ND | 6.0 |
| 7 | 89.9 | 84 | 7.6 |
| 8 | 86.7 | 91 | 8.8 |

### Example 7

Three preparations were done following the same protocol set forth in Example 6, except that dilute Tc-99 pertechnetate was added to the Tc-99m in order to carry more Tc mass.

One prepartion was a control (prep pH 7) and the two other prepartions contained an additional 5 nanomoles of Tc-99 (since 1 mL TcO₄⁻ is used, the prepartion woudl be made with 5 µM Tc, the highest usually eluted from a Mo-99/Tc-99m generator). Amoung these preparations, one was done at 50°C for 30 min instead of the 100°C (boiling water bath) for 5 min.

### Example 7: Results

| Preparation | RCY | Recovery (%) | Measured pH |
|---|---|---|---|
| control | 89.9 | 89 | 7.3 |
| 100°C, 5 min | 70.2 | 83 | 7.6 |
| 50°C, 30 min | 25.4 | 79 | ND |

The results above clearly indicate that a pyridine ligands having a THP protecting group can be labelled in a wide rage of pH conditions ranging from acidic to basic. The preparations made with additional Tc-99 showed somewhat reduced kinetics but still provided good yield of product. This precludes the possibility that the results could be explained by radiolabelling of an impurity of the ligand. Radiolabelling was shown to occur even at reduced temperature.

Based of the above results, it is believed that the pyridine ligand plays a major role in the radiolabelling properties. In addition, it is believed that the THP protecting group, previously thought to be an acid cleavable protector can be used to protect the ligand and allow excellent radiolabelling of the product, even under neutral and basic conditions.

The radiolabelled somatostatin compound of the present invention are described in still greater detail in the illustrative examples which follow.

### Example 8

A solution of somatostatin, or derivatives thereof, (0.01 mmol) in 2 mL of carbonate/bicarbonate buffer at pH 8.5 ± 0.5 is treated with a solution of 0.1 mmol of the ligand in Figure 1 (wherein q=4, and Z is succinimidoloxycarbonyl) in dimethylformamide (0.5 mL) and the entire mixture is kept at room temperature for 2 hours. The mixture is then diluted with water (2.5 mL) and dialyzed extensively against water. After dialysis, the solution is lyophilized to give the desired somatostatin conjugate.

### Example 9

A mixture of gentisic acid (25 mg), inositol (10 mg), and the somatostatin, or derivatives thereof, conjugate (500 uL, 1 mg/mL in water) was treated with In-111 indium chloride in 0.05 M HCl. The solution was allowed to incubate at room temperature for about 30 minutes. The desired labelled peptide is separated from unreacted In-111 indium salts and other small molecular weight impurities by gel filtration chromatography (Sephadex G-50) using phosphine buffered physiological saline, (PBS), 0.15M NaCl as eluent.

After the somatostatin or a derivative thereof is prepared and labelled according to a procedure described above, the compound is used with a pharmaceutically acceptable carrier in a method of performing a diagnostic imaging procedure using a gamma camera or like device. This procedure involves injecting or administering, for example in the form of an injectable liquid, to a warm-blooded animal an effective amount of the present invention and then exposing the warm-blooded animal to an imaging procedure using a suitable detector, e.g. a gamma camera. Images are obtained by recording emitted radiation of tissue or the pathological process in which the radioactive peptide has been incorporated, which in the present case are thrombus, thereby imaging thrombus in the body of the warm-blooded animal. Pharmaceutically acceptable carriers for either diagnostic or therapeutic use include those that are suitable for injection or administration such as aqueous buffer solutions, e.g. tris (hydroxymethyl)aminomethane (and its salts), phosphate, citrate, bicarbonate, etc., sterile water for injection, physiological saline, and balanced ionic solutions containing chloride and/or bicarbonate salts of normal blood plasma cations such as Ca²⁺, Na⁺, K⁺ and Mg²⁺. Other buffer solutions are described in Remington's Practice of Pharmacy, 11th edition, for example on page 170. The carriers may contain a chelating agent, e.g. a small amount of ethylenediaminetetraacidic acid, calcium disodium salt, or other pharmaceutically acceptable chelating agents.

The concentration of labeled peptide and the pharmaceutically acceptable carrier, for example in an aqueous medium, varies with the particular field of use. A sufficient amount is present in the pharmaceutically acceptable carrier in the present invention when satisfactory visualization of the tumor is achievable or therapeutic results are achievable.

The composition is administered to the warm-blooded animals so that the composition remains in the living animal for about six to seven hours, although shorter and longer residence periods are normally acceptable.

The radiolabelled somatostatin compounds of the present invention or somatostatin derivatives thereof, prepared as described herein, provide means of in vivo diagnostic imaging of tumors or therapeutic treatment of tumors which provides many advantages over prior known procedures for targeting the particular tumors of choice.

After consideration of the above specification, it will be appreciated that many improvements and modifications in the details may be made without departing from the scope of the invention. It is to be understood, therefore, that the invention is in no way limited, except as defined by the appended claims.

## Claims

1. Somatostatin or a molecule having somatostatin receptor specificity conjugated with a phenolic ligand having the general structure 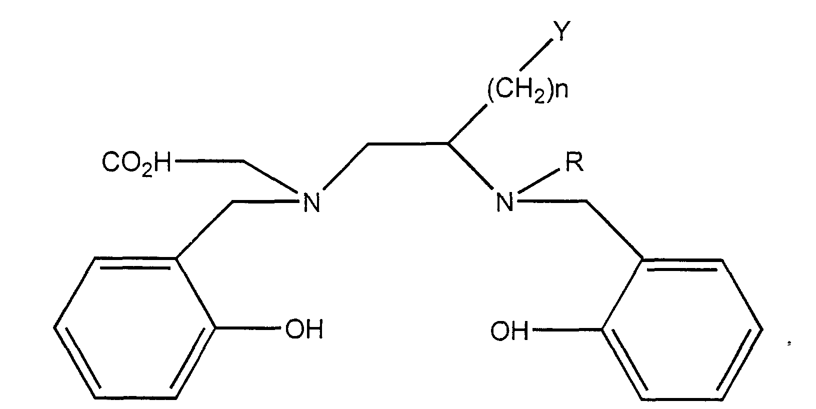 wherein n is a whole number less than eleven; Y is a coupling moiety selected from the group consisting of carboxyl, amino, isocyanate, isothiocyanate, imidate, malaeimide, chlorocarbonyl, chlorosulfonyl, succinimidyloxycarbonyl, haloacetyl and C₁₋₁₀ N-alkoxycarbamoyl; and R is hydrogen or a C₁₋₁₀ alkyl.

2. Somatostatin or a molecule having somatostatin receptor specificity conjugated with a metal complex having the general structure 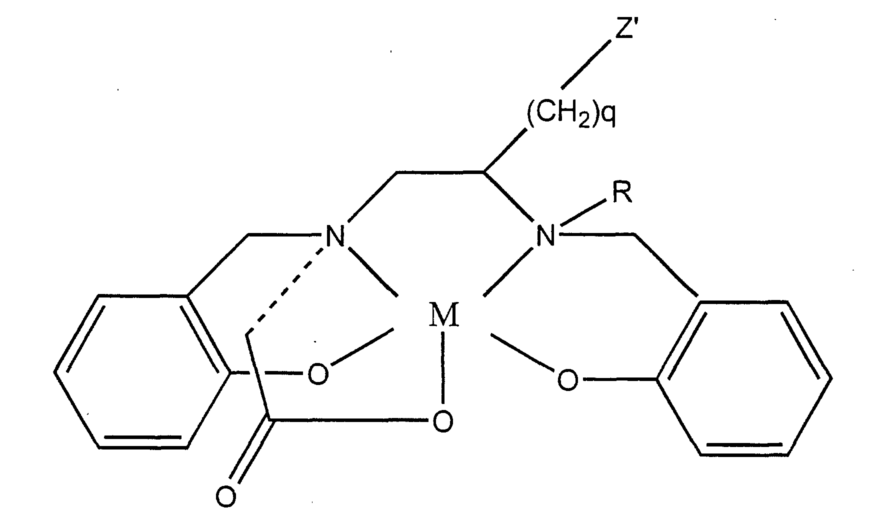 wherein q is a whole number less than eleven; wherein Z' is a coupling moiety selected from the group consisting of carboxyl, amino, isocyanate, isothiocyanate, imidate, malaeimide, chlorocarbonyl, chlorosulfonyl, succinimidyloxycarbonyl, haloacetyl and C₁₋₁₀ N-alkoxycarbamoyl; R is selected from the group consisting of hydrogen, and C₁₋₁₀ alkyl; and M is technetium, rhenium, indium, yttrium, gallium, samarium, holmium, copper or cobalt.

## Patentansprüche

1. Somatostatin oder Molekül mit Somatostatinrezeptorspezifität, das mit einem phenolischen Liganden konjugiert ist, mit der allgemeinen Struktur 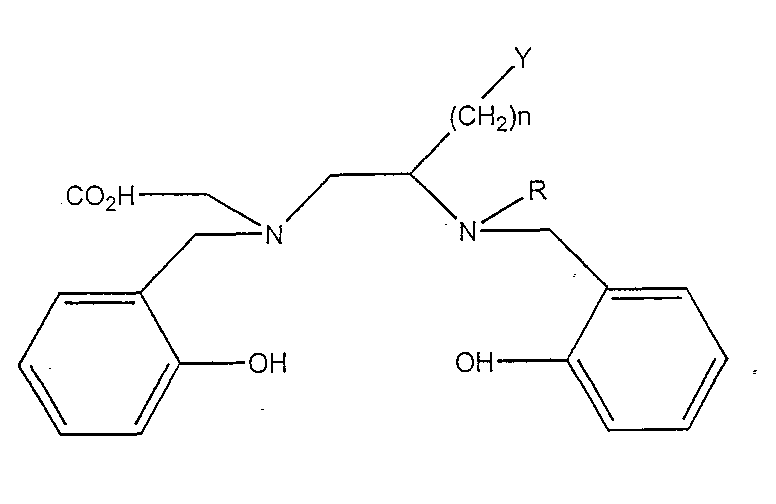 worin n eine ganze Zahl, kleiner als elf ist; Y eine Verknüpfungseinheit ist, die aus der aus Carboxyl, Amino, Isocyanat, Isothiocyanat, Imidat, Malaeimid, Chlorocarbonyl, Chlorosulfonyl, Succinimidyloxycarbonyl, Haloacetyl und C₁₋₁₀ N-Alkoxycarbamoyl bestehenden Gruppe gewählt ist; und R Wasserstoff oder ein C₁₋₁₀Alkyl ist.

2. Somatostatin oder Molekül mit Somatostatinrezeptorspezifität, das mit einem Metallkomplex konjugiert ist, mit der allgemeinen Struktur 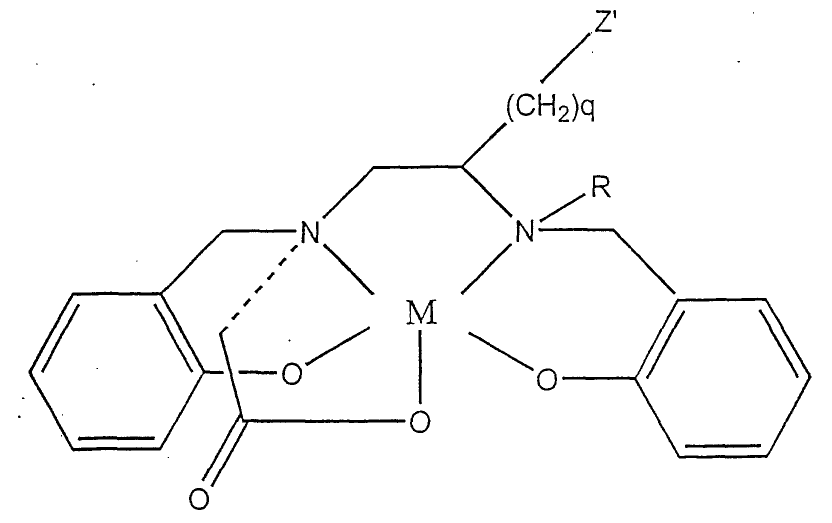 worin q eine ganze Zahl, kleiner als elf ist; worin Z' eine Verknüpfungseinheit ist, die aus der aus Carboxyl, Amino, Isocyanat, Isothiocyanat, Imidat, Malaeimid, Chlorocarbonyl, Chlorosulfonyl, Succinimidyloxycarbonyl, Haloacetyl und C₁₋₁₀ N-Alkoxycarbamoyl gebildeten Gruppe gewählt ist; R aus der aus Wasserstoff und C₁₋₁₀Alkyl bestehenden Gruppe gewählt ist; und M Technetium, Rhenium, Indium, Yttrium, Gallium, Samarium, Holmium, Kupfer oder Kobalt ist.

## Revendications

1. Somatostatine ou molécule ayant une spécificité de récepteur de somatostatine conjuguée avec un ligand phénolique ayant la structure générale : 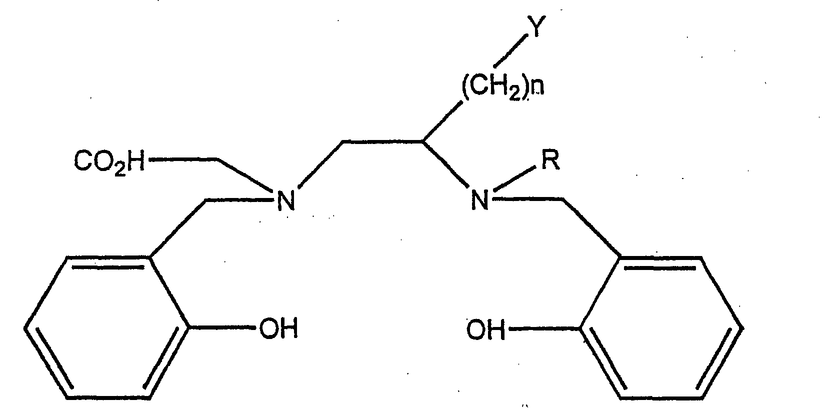 dans laquelle n est un nombre entier inférieur à 11, Y est un radical de couplage choisi dans le groupe constitué des groupes carboxyle, amino, isocyanate, isothiocyanate, imidate, maléimide, chlorocarbonyle, chlorosulfonyle, succinimidyloxycarbonyle, haloacétyle et N-alcoxycarbamoyle en C₁-C₁₀; et R est de l'hydrogène ou un groupe alkyle en C₁-C₁₀.

2. Somatostatine ou molécule ayant une spécificité de récepteur de somatostatine conjuguée avec un complexe métallique ayant la structure générale : 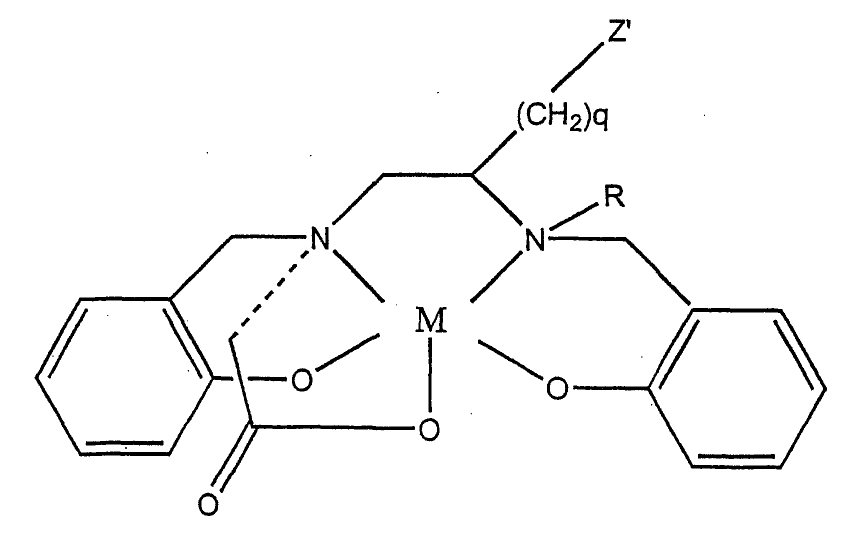 dans laquelle q est un nombre entier inférieur à 11 ; Z' est un radical de couplage choisi dans le groupe constitué des groupes carboxyle, amino, isocyanate, isothiocyanate, imidate, maléimide, chlorocarbonyle, chlorosulfonyle, succinimidyloxycarbonyle, haloacétyle et N-alcoxycarbamoyle en C₁-C₁₀; R est choisi dans le groupe constitué de l'hydrogène et d'un groupe alkyle en C₁-C₁₀; et M est le technétium, le rhénium, l'indium, l'yttrium, le gallium, le samarium, l'holmium, le cuivre ou le cobalt.
